# EUROPEAN PATENT SPECIFICATION

(11) **EP 4 507 572 B1**
(45) Date of publication and mention of the grant of the patent: **21.01.2026**
(21) Application number: 23722062.9
(22) Date of filing: 11.04.2023
(51) Int. Cl.: A61B 5/07, A61B 5/00, A61B 17/70, A61B 17/00, A61F 2/00

(54) **SPINAL IMPLANTS WITH ACTIVE SENSING CAPABILITIES**
WIRBELSÄULENIMPLANTATE MIT AKTIVEN MESSKAPAZITÄTEN
IMPLANTS RACHIDIENS À CAPACITÉS DE DÉTECTION ACTIVES

(30) Priority: 12.04.2022 US 202263329982 P; 19.12.2022 US 202218068140
(43) Date of publication of application: 19.02.2025
(73) Proprietor: Warsaw Orthopedic, Inc., Warsaw, IN 46582 (US)
(72) Inventor: METCALF, Newton H., Memphis, Tennessee 38111 (US); SIBY KURIAN, Arjun, Memphis, Tennessee 38103 (US); DACE, Mark C., Collierville, Tennessee 38017 (US); ALLEY, Steven C., Germantown, Tennessee 38139 (US)
(74) Representative: Viering, Jentschura & Partner mbB Patent- und Rechtsanwälte
(86) International application number: PCT/IB2023/053707
(87) International publication number: WO 2023/199226

(56) References cited:
- US-A1- 2017 007 420
- US-A1- 2020 069 247
- US-A1- 2020 297 513

## Description

### FIELD

The present disclosure generally relates to mechanical and electrical sensor assemblies and antenna designs for implant devices, and more particularly to spinal implant systems which may be used to treat various spinal disorders.

### BACKGROUND

Treatment of spinal disorders, such as degenerative disc disease, disc herniations, scoliosis or other curvature abnormalities, and fractures, often requires surgical treatments. For example, spinal fusion may be used to limit motion between vertebral members. As another example, implants may be used to preserve motion between vertebral members.

Surgical treatment typically involves the use of implants and longitudinal members, such as spinal rods. Implants may be disposed between two vertebral members for supporting and/or repositioning the vertebral members. Implants may also be used to facilitate a fusion process between a superior vertebrae and an inferior vertebrae. Longitudinal members may be attached to the exterior of two or more vertebral members to assist with the treatment of a spinal disorder. Longitudinal members may provide a stable, rigid column that helps bones to fuse, and may redirect stresses over a wider area away from a damaged or defective region. Also, rigid longitudinal members may help in spinal alignment.

Screw assemblies may be used to connect a longitudinal member to a vertebral member. A screw assembly may include a pedicle screw, hook, tulip bulb connector or other type of receiver, and a set screw, among other components. A pedicle screw can be placed in, above and/or below vertebral members that were fused, and a longitudinal member can be used to connect the pedicle screws which inhibits or controls movement. A set screw can be used to secure the connection of a longitudinal member and a pedicle screw, hook, or other connector. However, the connection force and continued integrity of the connection between a longitudinal member and a pedicle screw or other connector can be challenging to monitor during and after implantation. In addition, it is difficult to monitor that an appropriate force is maintained between a set screw and a longitudinal member. Conventional spinal implants, load assemblies, and/or screw assemblies are not capable of sensing and transmitting the connection force between a longitudinal rod and a pedicle screw installed within a patient. Conventional spinal implants are not capable of sensing the stress / strain applied to the spinal implant, by, e.g., the pressure between adjacent vertebrae and/or the pressure applied by an adjacent pedicle screw, longitudinal rod, etc. Furthermore, they cannot continuously monitor and maintain a secure connection on relatively long-time frames.

From e.g. US 2017 / 0 007 420 A1 a load sensing spinal implant is known, comprising: an interbody cage extending in a longitudinal direction from a proximal end to a distal end and in a widthwise direction from a first lateral end to a second lateral end; an electronics portion for supporting an electronics assembly and a battery therein; at least one antenna and in electrical communication with the electronics assembly; and at least one strain gauge configured to detect a localized force experienced by the interbody cage and being in electrical communication with the electronics assembly, wherein the at least one antenna is configured to transmit information received from the at least one strain gauge to an external device.

A further load sensing spinal implant is known from e.g. US 2020 / 0 297 513 A1.

### SUMMARY

The techniques of this disclosure generally relate to spinal implants having various sensors for communicating attributes about the spinal implants when installed in patient anatomy to an external reader.

The present invention provides a load sensing spinal implant with the features according to claim 1. Further preferred embodiments of the implant are described in the dependent claims.

The electronics assembly may be disposed on the side of the cage, a distal end of the cage, or inside a graft window of the cage.

Disclosed implants may include an overmold portion surrounding the electronics portion thereby forming a hermetic seal. In various embodiments, at least one antenna may be in electrical communication with the electronics assembly and have a size and shape that generally corresponds to a size and shape of at least one sidewall of the graft window. In disclosed embodiments, at least one strain gauge may be configured to detect a localized force experienced by the interbody cage and be in electrical communication with the electronics assembly. In at least some embodiments, the at least one antenna is configured to transmit information received from the at least one strain gauge to an external device.

In at least some embodiments, the interbody cage may include an exposed cavity at a distal end thereof having a curved sidewall, and the electronics portion is disposed inside of the exposed cavity. Additionally, the housing may conform to the curved sidewall, and the at least one strain gauge may be disposed inside of the housing and have a geometry corresponding to the curved sidewall.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a first embodiment of a spinal implant.
FIG. 2 is a top-down view of the embodiment of FIG. 1.
FIG. 3 is an exploded parts view of the embodiment of FIG. 1.
FIG. 4 is a first partial parts perspective view of the embodiment of FIG. 1.
FIG. 5 is a second partial parts perspective view of the embodiment of FIG. 1.
FIG. 6 is a perspective view of a second embodiment of a spinal implant.
FIG. 7 is an exploded parts view of the embodiment of FIG. 6.
FIG. 8 is a first perspective view of a third embodiment of a spinal implant.
FIG. 9 is a second perspective view of the embodiment of FIG. 8.
FIG. 10 is a first exploded parts view of the embodiment of FIG. 8.
FIG. 11 is a second exploded parts view of the embodiment of FIG. 8.
FIG. 12 is a first side view of the embodiment of FIG. 8.
FIG. 13 is a second side view of the embodiment of FIG. 8.
FIG. 14 is a first perspective view of a fourth embodiment of a spinal implant.
FIG. 15 is a second perspective view of the embodiment of FIG. 14.
FIG. 16 is an exploded parts view of the embodiment of FIG. 14.
FIG. 17 is a perspective view of a fifth embodiment of a spinal implant.
FIG. 18 is a top-down view of the embodiment of FIG. 17.
FIG. 19 is a side view of a microelectronics sub-assembly and antenna for use with the embodiment of FIG. 17.
FIG. 20 is a perspective view of the embodiment of FIG. 17 with the microelectronics sub-assembly and antenna of FIG. 18 removed.
FIG. 21 is an exploded parts view of the embodiment of FIG. 17.
FIG. 22 is an exploded parts view of the microelectronics sub-assembly and antenna for use with the embodiment of FIG. 17.

### DETAILED DESCRIPTION

Embodiments of the present disclosure relate generally, for example, to spinal implant systems with active sensing, microelectronics, and communication abilities. Embodiments of the devices and methods are described below with reference to the Figures.

The following discussion omits or only briefly describes certain components, features and functionality related to medical implants, installation tools, and associated surgical techniques, which are apparent to those of ordinary skill in the art. It is noted that various embodiments are described in detail with reference to the drawings, in which like reference numerals represent like parts and assemblies throughout the several views, where possible. Reference to various embodiments does not limit the scope of the claims appended hereto because the embodiments are examples of the inventive concepts described herein. Additionally, any example(s) set forth in this specification are intended to be non-limiting and set forth some of the many possible embodiments applicable to the appended claims. Further, particular features described herein can be used in combination with other described features in each of the various possible combinations and permutations unless the context or other statements clearly indicate otherwise.

Terms such as "same," "equal," "planar," "coplanar," "parallel," "perpendicular," etc. as used herein are intended to encompass a meaning of exactly the same while also including variations that may occur, for example, due to manufacturing processes. The term "substantially" may be used herein to emphasize this meaning, particularly when the described embodiment has the same or nearly the same functionality or characteristic, unless the context or other statements clearly indicate otherwise. The term "about" may encompass a meaning of being +/- 10% of the stated value.

Referring to the disclosed embodiments generally, various vertebral pedicle screw systems are disclosed. The components of the vertebral pedicle screw systems can be fabricated from biologically acceptable materials suitable for medical applications, including metals, synthetic polymers, ceramics and bone material and/or their composites. For example, the components, individually or collectively, can be fabricated from materials such as stainless steel alloys, commercially pure titanium, titanium alloys, Grade 5 titanium, super-elastic titanium alloys, cobalt-chrome alloys, superelastic metallic alloys (e.g., Nitinol, super elasto-plastic metals, such as GUM METAL^{®}), ceramics and composites thereof such as calcium phosphate (e.g., SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO4 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe, polylactic acid or polylactide and their combinations.

Various components of the vertebral implant system may be formed or constructed with material composites, including the above materials, to achieve various desired characteristics such as strength, rigidity, elasticity, compliance, biomechanical performance, durability and radiolucency or imaging preference. The components of the present vertebral pedicle screw system, individually or collectively, may also be fabricated from a heterogeneous material such as a combination of two or more of the above-described materials. The components of the vertebral implant system may be monolithically formed, integrally connected or include fastening elements and/or instruments, as described herein. The components of the vertebral implant system may be formed using a variety of subtractive and additive manufacturing techniques, including, but not limited to machining, milling, extruding, molding, 3D-printing, sintering, coating, vapor deposition, and laser/beam melting.

Furthermore, various components of the vertebral implant system may be coated or treated with a variety of additives or coatings to improve biocompatibility, bone growth promotion or other features. Various embodiments and components may be coated with a ceramic, titanium, and/or other biocompatible material to provide surface texturing at (a) the macro scale, (b) the micro scale, and/or (c) the nano scale, for example. Similarly, components may undergo a subtractive manufacturing process such as, for example, grit blasting and acid etching, providing for surface texturing configured to facilitate osseointegration and cellular attachment and osteoblast maturation. Example surface texturing of additive and subtractive manufacturing processes may comprise (a) macro-scale structural features having a maximum peak-to-valley height of about 40 microns to about 500 microns, (b) micro-scale structural features having a maximum peak-to-valley height of about 2 microns to about 40 microns, and/or (c) nano-scale structural features having a maximum peak-to-valley height of about 0.05 microns to about 5 microns. In various embodiments, the three types of structural features may be overlapping with one another. Additionally, such surface texturing may be applied to any surface, e.g., both external exposed facing surfaces of components and internal non exposed surfaces of components. Further discussion regarding relevant surface texturing and coatings is described in, for example, U.S. Pat. No. 11,096,796, titled Interbody spinal implant having a roughened surface topography on one or more internal surfaces, and filed on March, 4, 2013. Accordingly, it shall be understood that any of the described coating and texturing processes of U.S. Pat. No. 11,096,796, may be applied to any component of the various embodiments disclosed herein, e.g., the exposed surfaces and internal surfaces. Another example technique for manufacturing an orthopedic implant having surfaces with osteoinducting roughness features including micro-scale structures and nano-scale structures is disclosed in U.S. Pat. No. 10,821,000. Additionally, an example of a commercially available product may be the Adaptix^{™} Interbody System sold by Medtronic Spine and comprising a titanium cage made with Titan nanoLOCK^{™}.

The disclosed implant systems may be employed, for example, with a minimally invasive procedure, including percutaneous techniques, mini-open and open surgical techniques to deliver and introduce instrumentation and/or one or more spinal implants at a surgical site within a body of a patient, for example, a section of a spine. In some embodiments, the vertebral implant system may be employed with surgical procedures, as described herein, and/or, for example, corpectomy, discectomy, fusion and/or fixation treatments that employ spinal implants to restore the mechanical support function of vertebrae. In some embodiments, the implant system may be employed with surgical approaches, including but not limited to: anterior lumbar interbody fusion (ALIF), direct lateral interbody fusion (DLIF), oblique lateral lumbar interbody fusion (OLLIF), oblique lateral interbody fusion (OLIF), transforaminal lumbar Interbody fusion (TLIF), posterior lumbar Interbody fusion (PLIF), various types of posterior or anterior fusion procedures, and any fusion procedure in any portion of the spinal column (sacral, lumbar, thoracic, and cervical). The surgical treatments described herein do not form part of the invention but are helpful in understanding the invention.

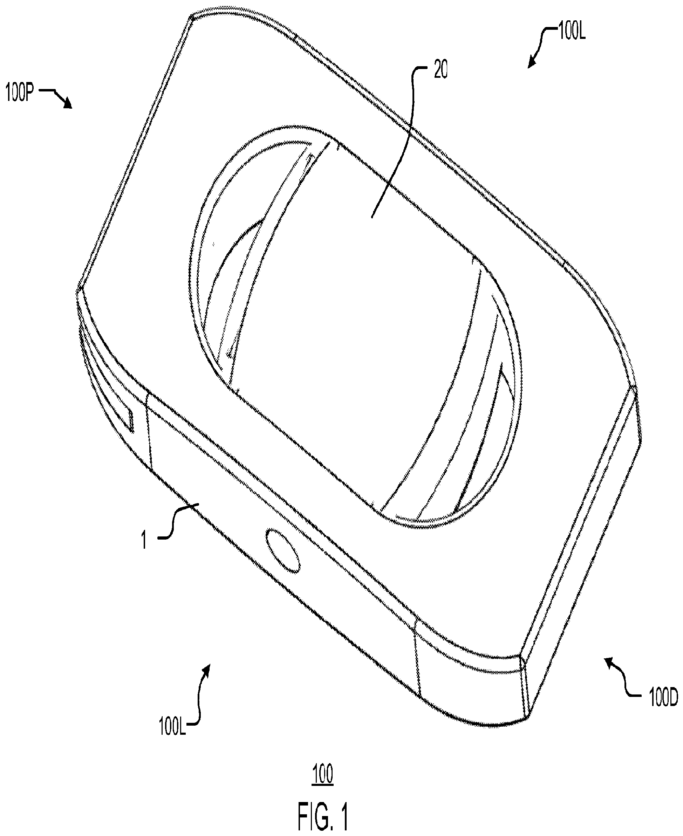

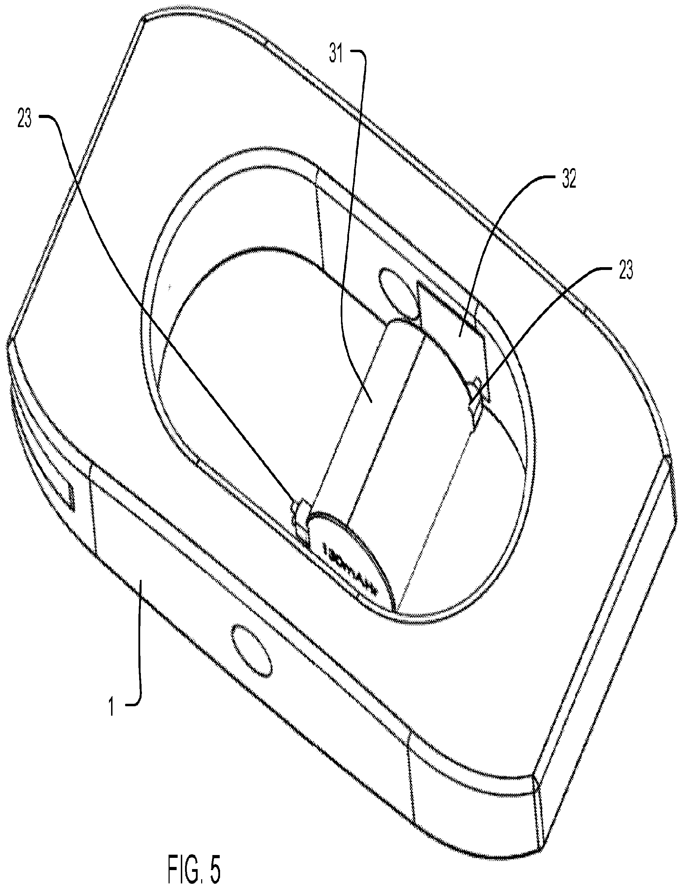

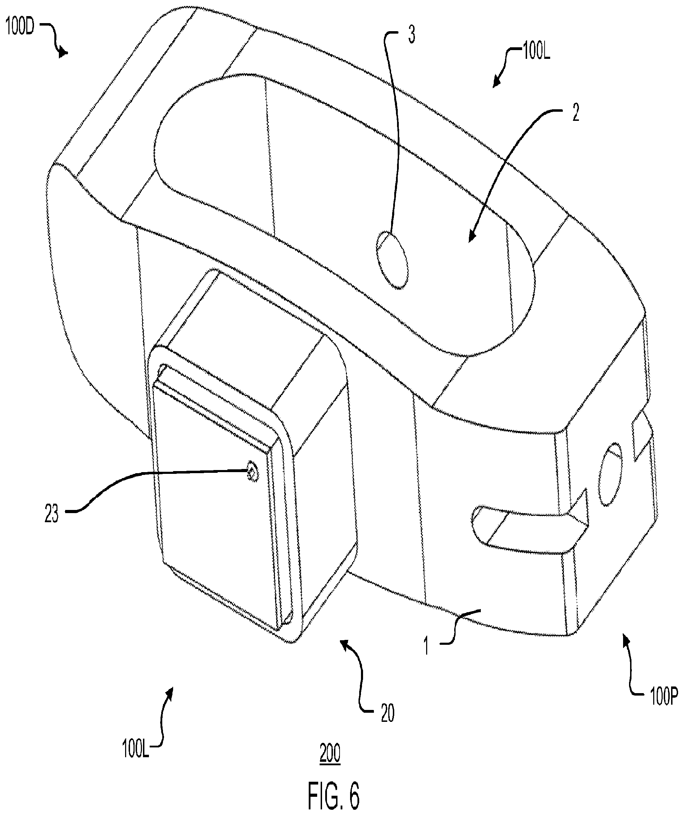

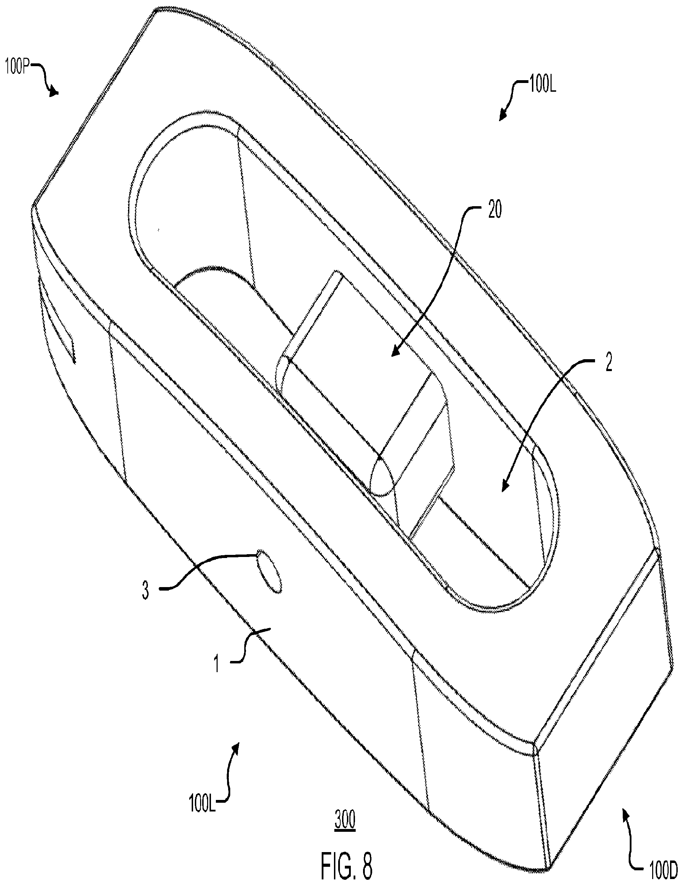

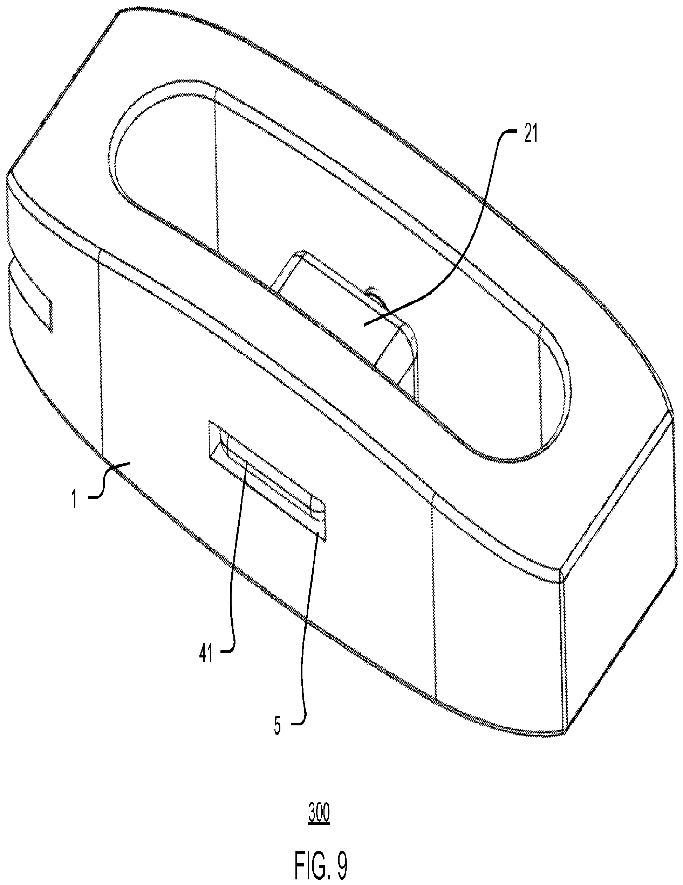

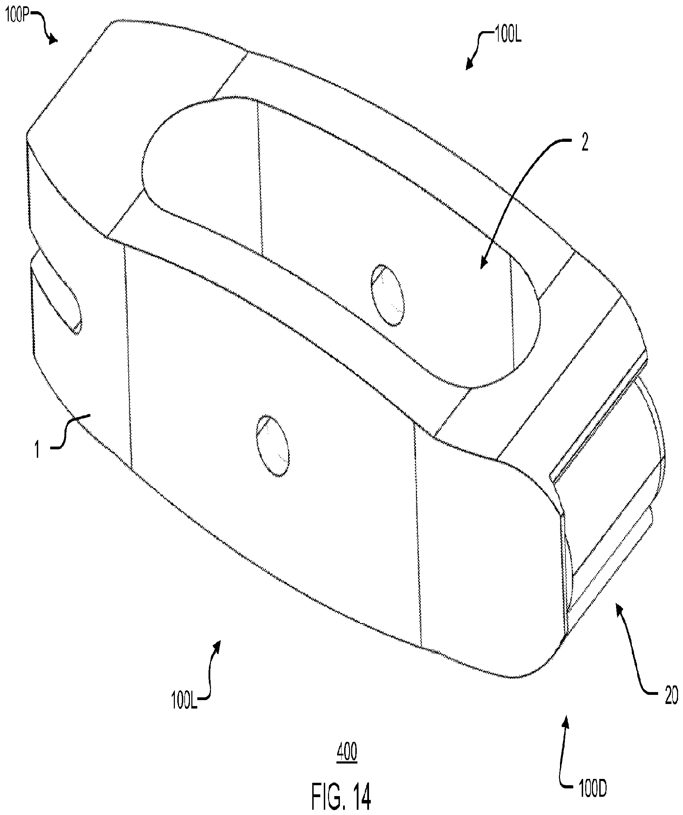

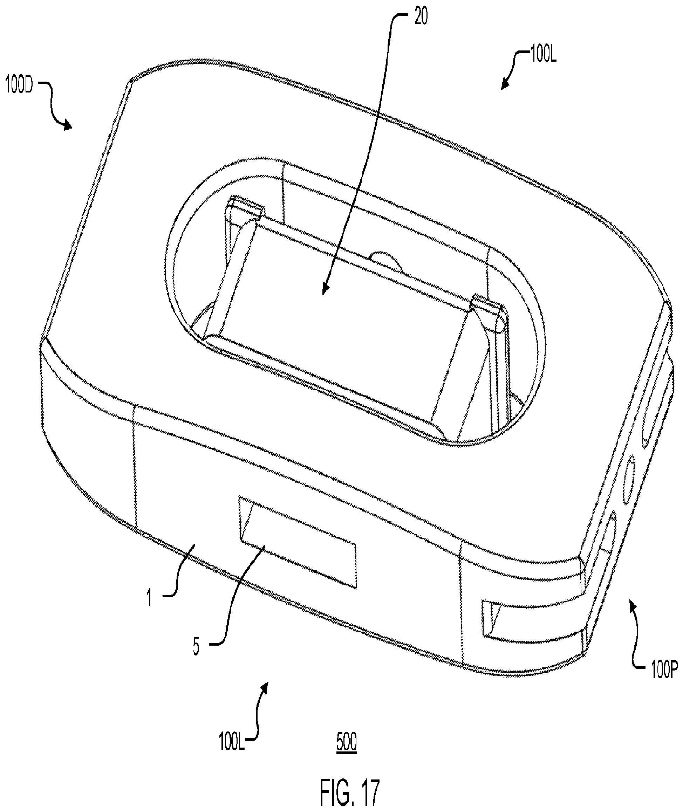

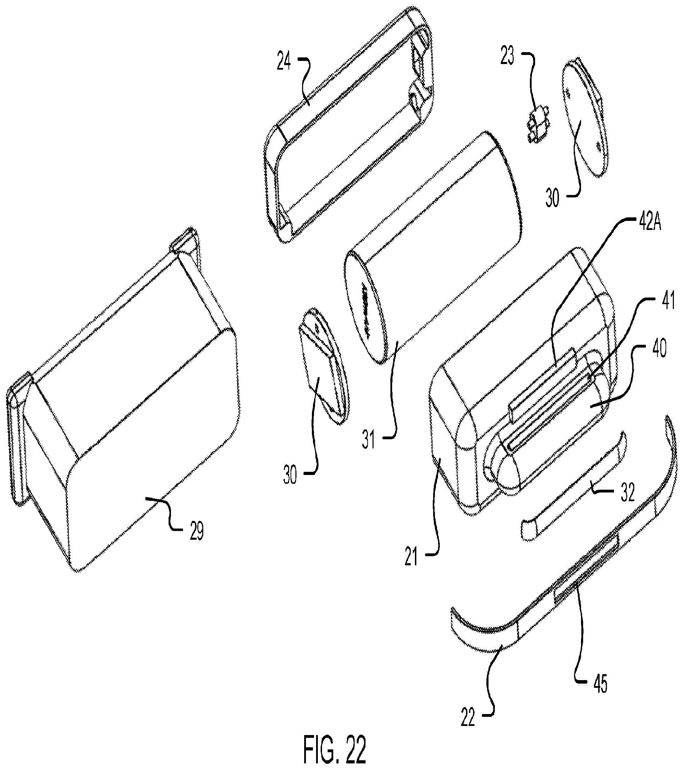

FIGS. 1-5 illustrate a first spinal implant system 100, FIGS. 6-7 illustrate a second spinal implant system 200, FIGS. 8-13 illustrate a third spinal implant system 300, FIGS. 14-16 illustrate a fourth spinal implant system 400, FIGS. 17-22 illustrate a fifth spinal implant system 500.

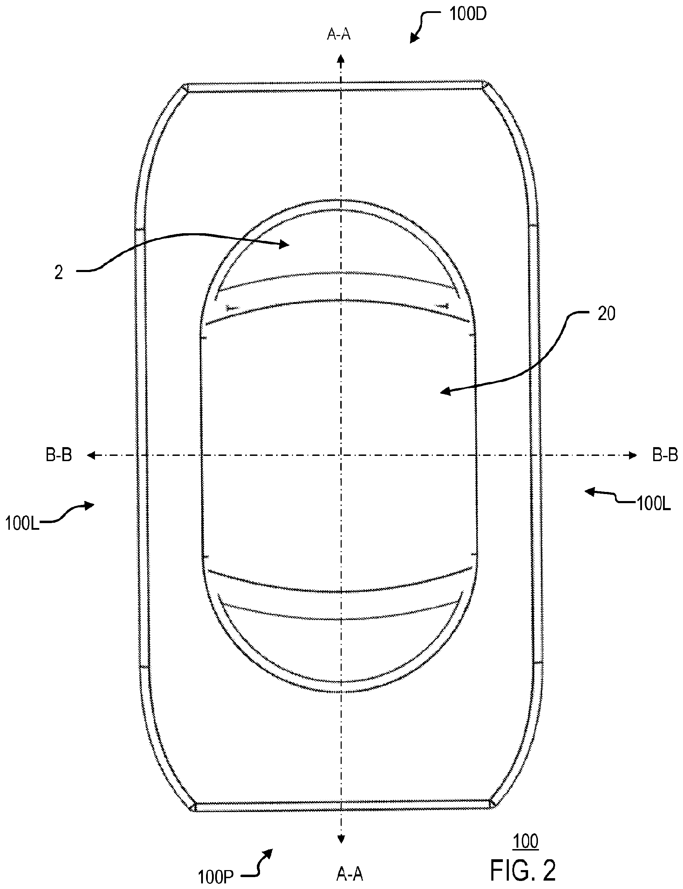

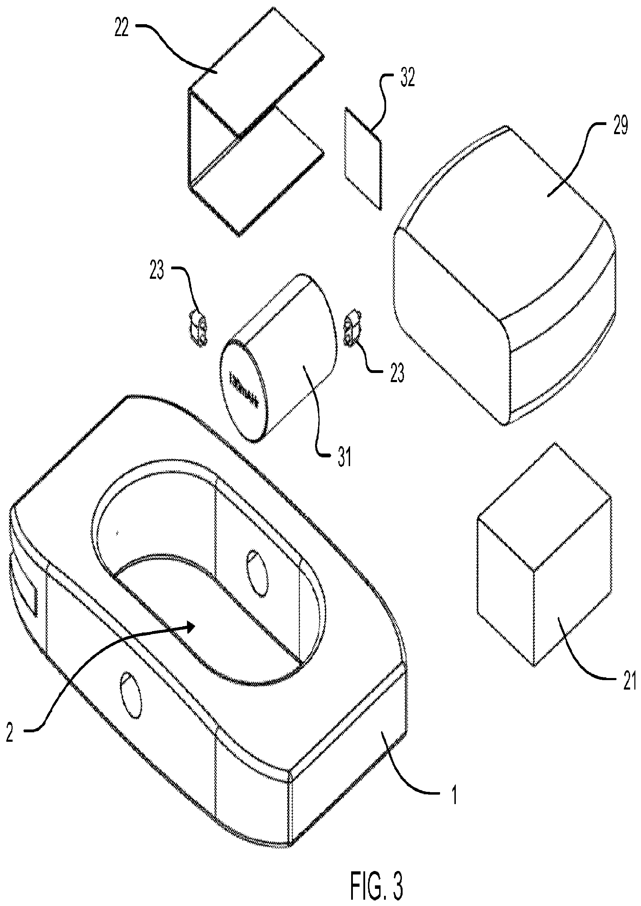

Referring generally to FIGS. 1-5, a spinal interbody implant system 100 is disclosed. As illustrated in FIGS. 1-2, system 100 may include an interbody cage 1 having a electronics portion 20 for supporting various electronic components and sensors therein as will be explained in further detail below. In various embodiments, interbody cage 1 may be integrally formed as a single monolithic component or interbody cage 1 may be an expandable cage with a superior endplate and an inferior endplate that may expand via an expansion mechanism. As seen in FIG. 2, cage 1 may extend in a longitudinal direction along axis A-A from a proximal end 100P to a distal end 100D. In various embodiments, the proximal end 100P may have various features for grasping of the cage 1 to facilitate insertion and the distal end 100D may generally serve as the leading edge during the insertion of cage 1 into a patient as would be understood by a person of ordinary skill in the art. Additionally, cage 1 may extend in a widthwise direction along axis B-B from a first lateral end 100L to a second lateral end 100L. In various embodiments the cage 1 may include a graft window 2 (see also FIG. 3) and the electronics portion 20 may be disposed therein.

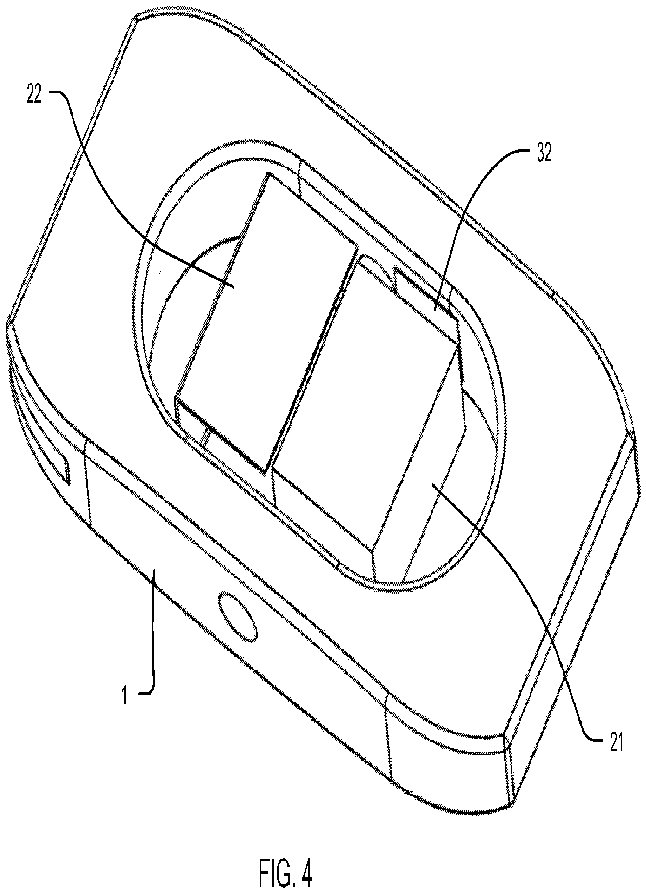

Referring to FIGS. 3-5 generally, various microelectronics may be disposed inside of the graft window 2 of cage 1. In this embodiment, the electronics portion 20 may include an electronics housing 21 and an antenna housing 22. The electronics housing 21 may house any electronics componentry explained herein, e.g., battery 31, printed circuit boards, sensors, etc. In the example embodiment, a battery 31 is disposed inside of the electronics housing 21 and a first pass-through connection 23 may extend through electronics housing 21 and place the battery 31 and an antenna disposed in the antenna housing 22 in electrical connection. Additionally, a second pass-through connection 23 may extend through electronics housing 21 and place the battery 31 and a sensor in electrical connection, e.g., strain gauge 32. strain gauge 32 may be disposed anywhere on cage 1 to actively sense stress and strain applied to cage 1, e.g., by a superior vertebrae and/or an inferior vertebrae. In the example embodiment, strain gauge 32 is disposed on an interior sidewall of graft window 2 (see FIG. 4) adjacent a medial portion of cage 1. In other embodiments, strain gauge 32 may be disposed on an interior sidewall of graft window 2 adjacent a proximal end 100P, a distal end 100D, or plural strain gauges 32 may be disposed in any of the aforementioned relative locations. Either one or both of the electronics housing 21, and the antenna housing 22 may be affixed to cage 1 by any means, e.g., a screw, pin, adhesive etc. In this embodiment, the electronics housing 21 and antenna housing 22 are placed inside of the graft window 2 and then an overmold 29 surrounds the electronics housing 21 and antenna housing 22. In this way, the overmold 29 may provide a hermetic seal to any component inside of the electronics housing 21 and antenna housing 22. However, an overmold 29 is not strictly necessary to provide a hermetic seal.

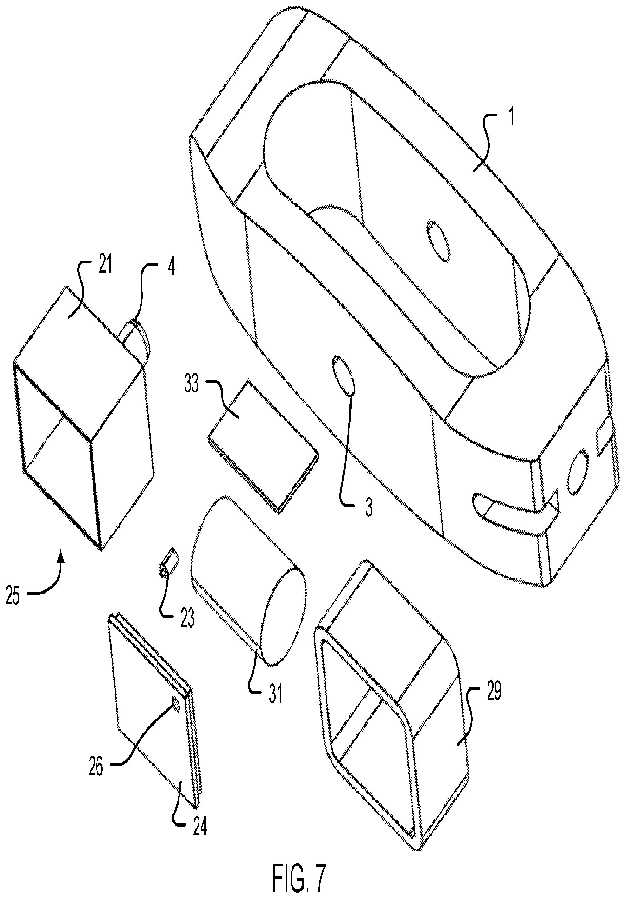

FIGS. 6-7 illustrate a second spinal implant system 200. Spinal implant system 200 may include the same, similar, and/or substantially the same components and functionality as explained above with respect to implant system 100 and vice versa. Accordingly, duplicative description will be omitted where possible and like numbering of parts will be maintained where possible. In this embodiment, cage 1 is a relatively thick cage including a graft window 2 and at least one fixation aperture 3. Additionally, in this embodiment the electronics portion 20 may be disposed on an external side surface of cage 1 rather than inside of the graft window 2. For example, the electronics portion 20 may be connected to an external and/or exposed lateral side surface 100L of implant 200 adjacent a medial portion thereof. Referring to the exploded parts view of FIG. 7, the electronics portion 20 may include a housing 21 that may define a cavity 25 therein for supporting various electronic components therein, e.g., the battery 31 and printed circuit board 33. In various embodiments, housing 21 may be attached, coupled, and or clipped onto cage 1 by inserting pin 4 through and/or into fixation aperture 3. In this way, housing 21 may be connected to any fixation aperture 3 disposed anywhere along an exposed surface of cage 1, including inside of the graft window 2. In various embodiments, cavity 25 of housing 21 may be hermetically sealed such that the electronics components therein will not harm a patient when the system 100 is installed within the human body. The battery 31 and circuit board 33 may be installed within the cavity 25 in any suitable way such that the circuit board 33, battery 31, are in electrical communication with a strain gauge 32 (not illustrated) and an antenna (not illustrated). For example, in this embodiment, implant system 200 includes a pass-through connection 23 which may be used to connect to a feed wire that attaches to an external sensor, antenna, electronics components, etc.

In the example embodiment, the cavity 25 may be sealed off by cover 24. Cover 24 may have a size and shape corresponding to an opening in housing 21 that exposes the cavity 25 therein. Due to the hermetically sealed nature of cavity 25, a pass-through connection 23 having suitable waterproof flanges may extend through an aperture 26 of cover 24 (see FIG. 7). In this way, the pass-through connection 23 may be electrically connected to a sensor and antenna external to the housing 21 while ensuring that a hermetic seal of the electronics components within housing 21 is possible. Although the antenna, strain gauge, and other sensors are not illustrated in FIGS. 6-7 it shall be appreciated that any of the example antennas and example sensors disclosed herein with reference to the other embodiments may be provided with the particular embodiment shown in FIGS. 6-7.

FIGS. 8-13 illustrate a third spinal implant system 300. Spinal implant system 300 may include the same, similar, and/or substantially the same components and functionality as explained above with respect to implant systems 100 and 200 and vice versa. Accordingly, duplicative description will be omitted where possible and like numbering of parts will be maintained where possible. In this embodiment, the electronics portion 20 is disposed inside of the graft window 2 and includes various geometrical protrusions that coordinate with geometrical indentations in the sidewall of cage 1 as will be explained in further detail below.

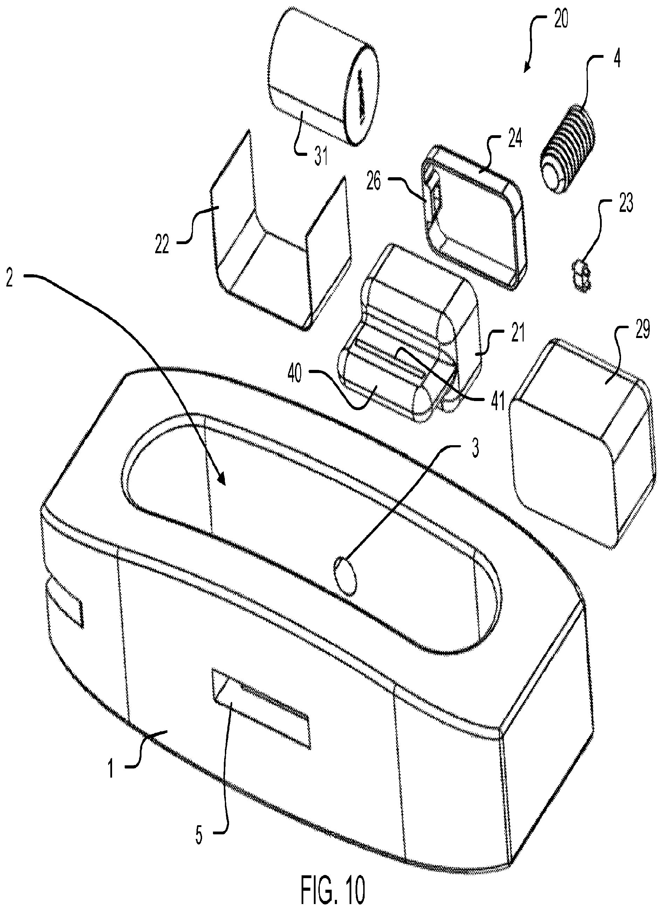

FIGS. 10-11 illustrate first and second exploded parts views of implant system 300. In the example embodiment, the housing 21 is surrounded by a molded antenna portion 22. In the example embodiment, molded antenna 22 has a U-like size and shape that generally corresponds to a size and shape of the housing 21 in at least one direction. In various embodiments, the molded antenna 22 may include an overmold portion such as an insulator that surrounds and/or encapsulates a conductive material such as copper for forming an antenna capable of communicating across various frequency bands. In various embodiments, the insulator material may be a thermoplastic material like Polyether ether ketone (PEEK). The conductive portion may be formed in any suitable pattern, e.g., as a 3D helix pattern, a slotted patch pattern, a 3D spiral pattern, a 2D spiral, and/or a meandered patch pattern. In various embodiments, the molded antenna 22 may include an overmold portion that surrounds and/or supports at least one type of antenna therein. Various antenna and communication types housed within molded antenna 22 may be, for example, MICS and BLE. As used herein, "MICS" may refer to the Medical Implant Communication System which may be a short-range communication technology that operates at a frequency from about 402 to 405 MHz. As used herein, "BLE" may refer to Bluetooth low energy communication standard. In some embodiments, at least one patch style antenna may be disposed within the antenna portion 22, for example an overmold or insulator may surround a MICS patch, a BLE patch, and/or a Dual-band electrically coupled loop antenna (ECLA) antenna.

Implant system 300 may include a cage 1 having a fixation aperture 3 in a first sidewall and a slotted aperture 5 (also referred to as a sensing slot 5) in a second sidewall. In various embodiments, the slotted aperture may have a geometry, size, and location configured to transfer localized stress and strain to a strain gauge 32 as will be explained in further detail below. In the example embodiment, the fixation aperture 3 and sensing slot 5 each extend through a corresponding sidewall of cage 1. For example, the fixation aperture and sensing slot 5 each extend through a respective sidewall of cage 1 thereby communicating with graft window 2 and the outside. In various embodiments, the electronics portion 20 may include a threaded post 4 and/or set screw that secures the electronics portion 20 to the fixation aperture 3 in a sidewall of the cage 1. In this embodiment, the electronics portion 20 may include a housing 21 defining a cavity 25 therein for housing various electronics components 30. The electronics components 30 may have great variability in the types of circuity and hardware due to the relatively large size of the housing 21 and cavity 25. Example electronics components may include a flexible circuit board providing an electrical connection between the battery 31, strain gauge 32, and the various other electronics components. A non-limiting list of example electronics components may include an Application Specific Integrated Controller (ASIC) 34, micro controller 35, a wake-up sensor, a memory storage, an impedance sensor, and a temperature sensor. In the example embodiment,

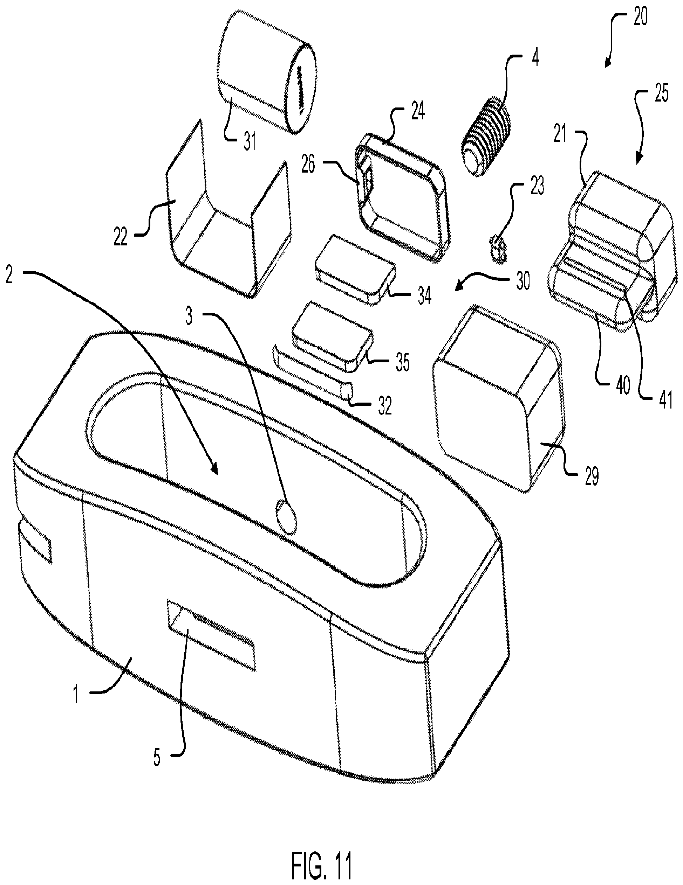

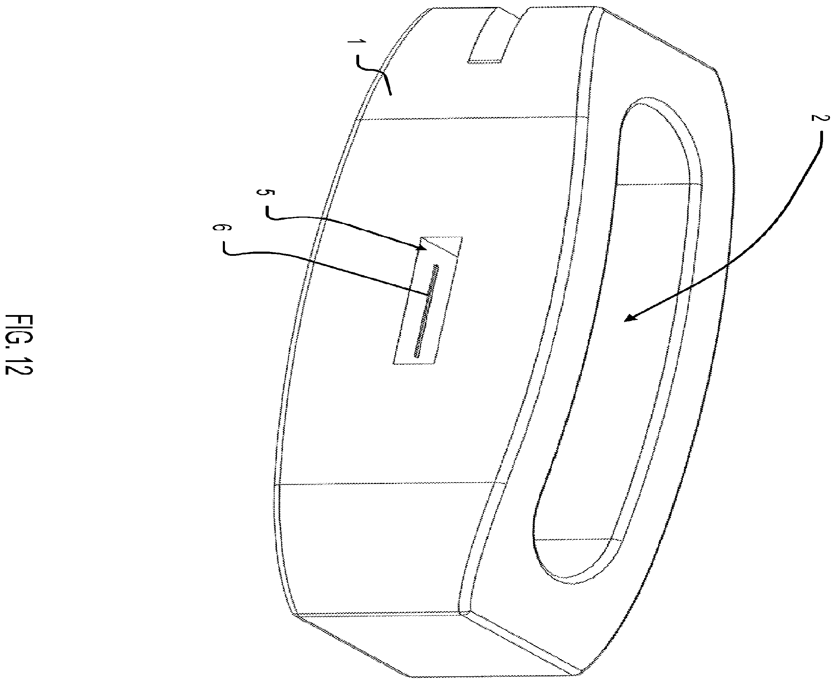

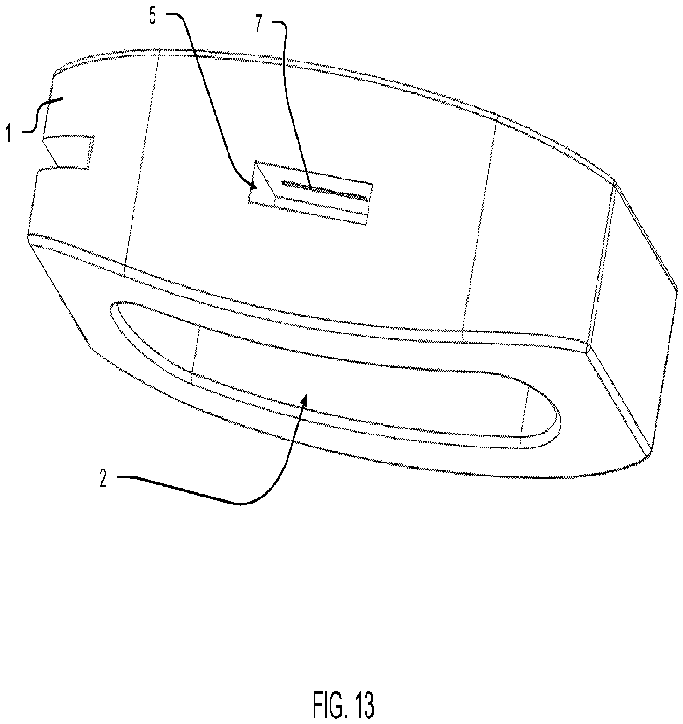

In various embodiments, the housing 21 may include a sensing protrusion 40 having a size and shape corresponding to a size and shape of the slotted aperture 5. Additionally, a strain gauge 32 may be disposed inside of the cavity 25 in the portion thereof corresponding to the sensing protrusion 40. For example, in this embodiment the strain gauge 32 may have a U-like shape corresponding in size and shape to the sensing protrusion 40 (see FIG. 11). Additionally, in various embodiments the sensing protrusion 40 may include at least one raised and/or indented rail portion 41 extending along an outside surface of the sensing protrusion 40. As seen best in FIGS. 8-9, the sensing protrusion 40 may extend into the slotted aperture 5 and be snug tight against the sidewalls of the slotted aperture 5. As seen best in FIGS. 12-13 the slotted aperture 5 may include an inferior indented slot 6 (see FIG. 12) and a superior indented slot 7 (see FIG. 13). The indented slots 6, 7 may correspond in size and shape to the rail portions 41 of the housing 21 such that the rail portions 41 may be disposed inside of slots 6, 7 when system 300 is fully assembled. This configuration of the slotted aperture 5, protruding portion 40, rail portion 41, and slots 6, 7 may be particularly advantageous at accurately transferring stress strain experienced by the cage 1 to the u-shaped strain gauge 32.

FIGS. 14-16 illustrate a fourth spinal implant system 400. Spinal implant system 400 may include the same, similar, and/or substantially the same components and functionality as explained above with respect to implant systems 100, 200, and 300 and vice versa. Accordingly, duplicative description will be omitted where possible and like numbering of parts will be maintained where possible.

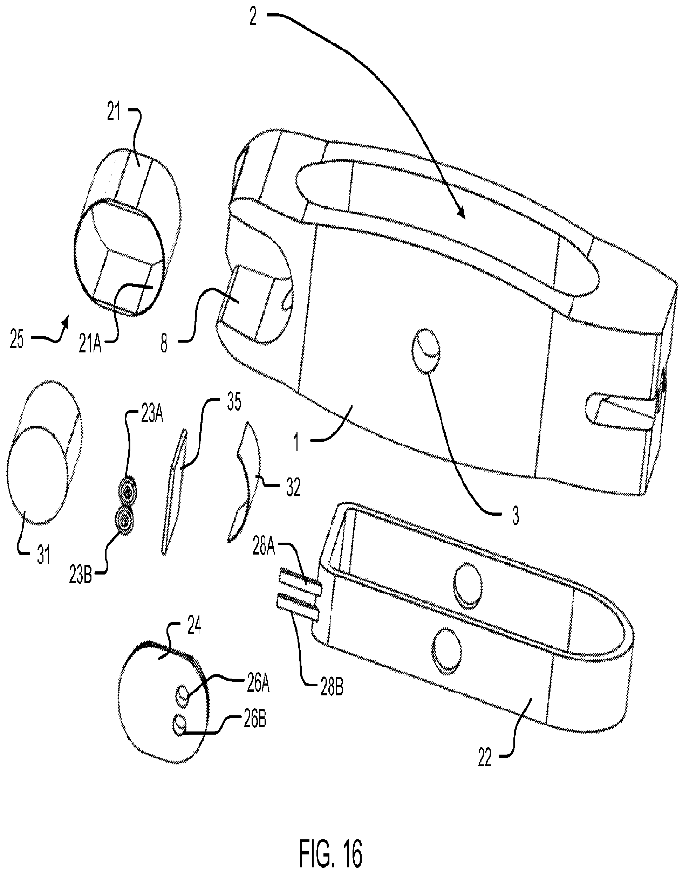

In various embodiments, the electronics portion 20 may be disposed in a cavity 8. In the illustrated example, the electronics portion 20 is disposed in a curved cavity 8 formed in the distal end 100D (see FIGS. 14-15). Referring to the exploded parts view of FIG. 16, it is shown that the cage 1 includes a curved cavity formed in a distal end thereof. Additionally, it is shown that the electronics portion 20 includes a housing 21 having a size and shape corresponding to the curved cavity 8 of cage 1. For example, housing 21 has an elongated cylindrical shape that corresponds in size, shape, and curvature to that of the curved cavity 8. Housing 21 may define a cavity 25 therein for storing various electronics components, e.g., battery 31, micro controller 35, strain gauge 32, etc. may be disposed in cavity 25. In this example, strain gauge 32 may be disposed on an interior sidewall 21A of housing 21. In this way, due to the corresponding geometry of housing 21 and cavity 8, as explained above, stress and strain experienced by cage 1 is accurately transferred to strain gauge 32. For example, the curved shape of housing 21, curved shape of strain gauge 32, and curved shape of cavity 8 all correspond to one another and therefore strain gauge 32 can accurately measure the stress and strain experienced by cage 1. This arrangement may be particularly advantageous for measuring stress and strain experienced at the distal end 100D of system 400, although it shall be understood that cavity 8 can be disposed in a sidewall at a medial position, a proximal position, a distal position. In various embodiments a plural number of cavities 8 and corresponding electronics portion 20 may be provided at any of the aforementioned regions of interest.

Once the various electronics components are positioned inside of cavity 25, cover 24 may be welded and/or adhered to housing 21 to seal the cavity 25 and thereby form a hermetic seal. In some embodiments, an overmold may be formed around the housing 21 (not illustrated). In the example embodiment, a first and second lead wire 23A, 23B may extend through apertures 26A, 26B of cover 24. The first and second lead wires 23A, 23B may each contact a corresponding first and second terminal 28A, 28B of antenna 22. In this embodiment, antenna 22 may have a size and shape generally corresponding to a centerline cage 1. In this example, a "centerline" may refer to a top-down view of cage 1 where a centerline traverses the oblong oval shape of cage 3 at equal distances from an interior perimeter defined by graft window 2 and an exterior perimeter defined by the outside sidewalls of cage 1. For example, antenna 22 has a size and shape that corresponds to a size and shape of cage 1 such that antenna 22 can be disposed inside of and/or surrounded by cage 1. In at least one embodiment, antenna 22 may be a metallic material such as copper and cage 1 may be an insulative material such as peek that is cast around antenna 22 by a mold in place process. In other embodiments, the general shape of cage 1 and material selection thereof may be chosen to amplify the transmission abilities of cage 1.

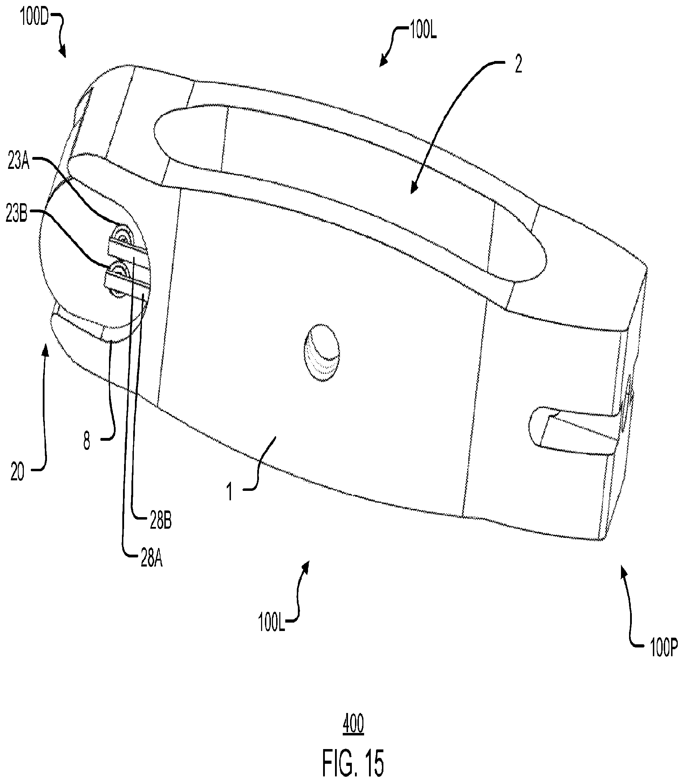

As seen best in FIG. 15, the first and second terminals 28A, 28B protrude through a sidewall of cage 1 to contact the first and second lead wires 23A, 23B thereby placing the antenna 22 in electrical communication with the electronics portion 20. In plan-view, the antenna 22 may be a relatively large, coiled shape having a perimeter that generally corresponds to a perimeter of cage 2. This may have the advantage of significantly increasing the transmissibility of information to and from implant system 400 at deeper depths within the tissue of human anatomy. In the example embodiment, in a top-down plan view, the perimeter of antenna 22 is slightly smaller than the perimeter of cage 1 on account of cage 1 generally surrounding antenna 22 (with the exception of terminals 28A, 28B extending through sidewall of cage 1).

FIGS. 17-22 illustrate a fifth spinal implant system 500. Spinal implant system 500 may include the same, similar, and/or substantially the same components and functionality as explained above with respect to implant systems 100, 200, 300, 400 and vice versa. Accordingly, duplicative description will be omitted where possible and like numbering of parts will be maintained where possible. Spinal implant system 500 may be similar in principle and functionality to spinal implant system 300 shown in FIGS. 8-13. For example, the electronics portion 20 may be disposed inside of the graft window 2 and include various geometrical protrusions that coordinate with geometrical indentations in the sidewall of cage 1 as will be explained in further detail below.

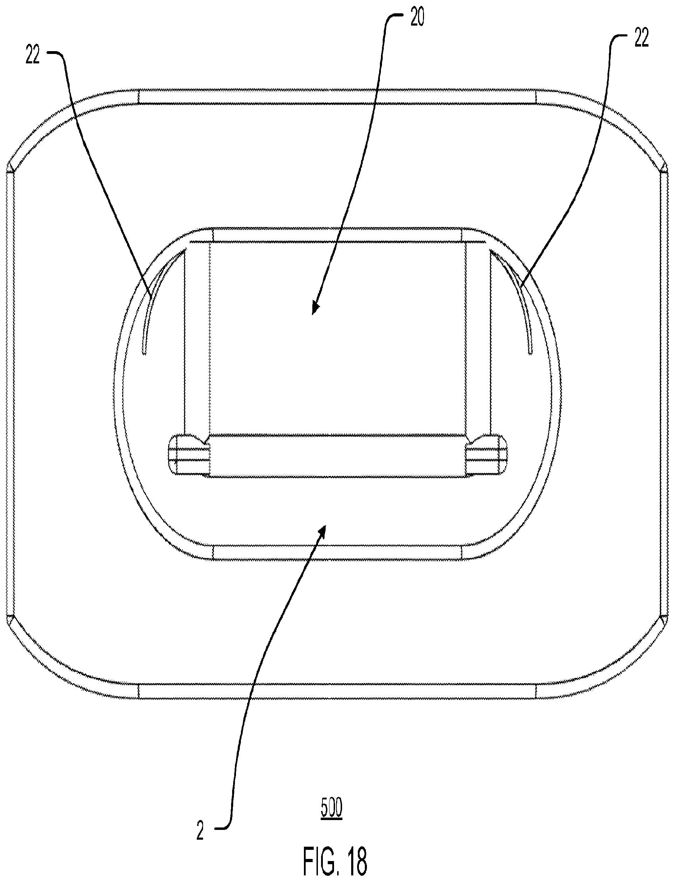

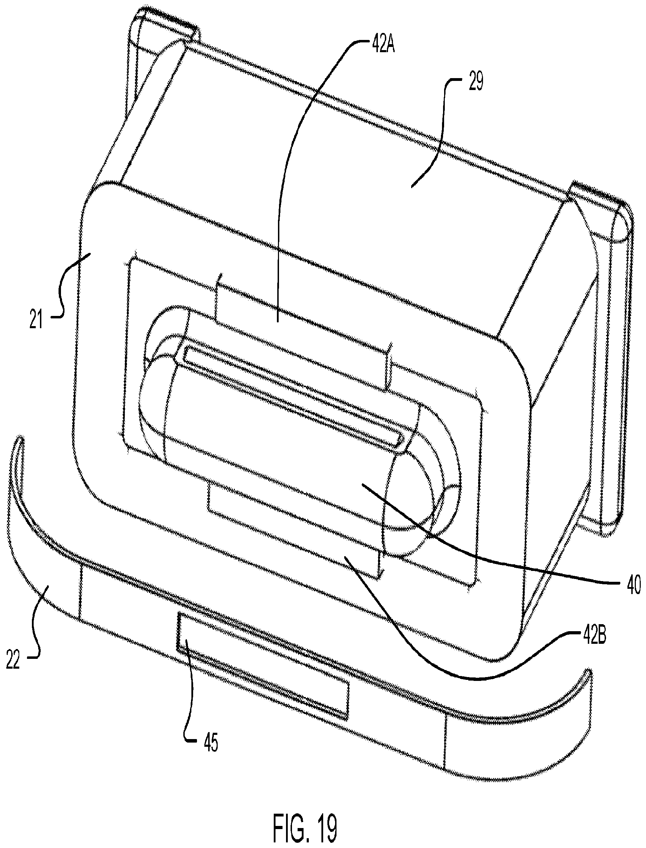

Referring to the top-down view in FIG. 18 and the partial exploded parts view in FIG. 19, the housing 21 is partly surrounded by a molded antenna portion 22 having a size and shape that generally corresponds to the interior perimeter of the graft window 2 (see top-down view in FIG. 18). In various embodiments, molded antenna 22 also has a U-like size and shape that generally corresponds to a size and shape of the housing 21 in at least one direction. Furthermore, as seen best in FIG. 19, antenna 22 may include an aperture 45 having a size and shape generally corresponding to a size and shape of protrusion 42B of housing 21.

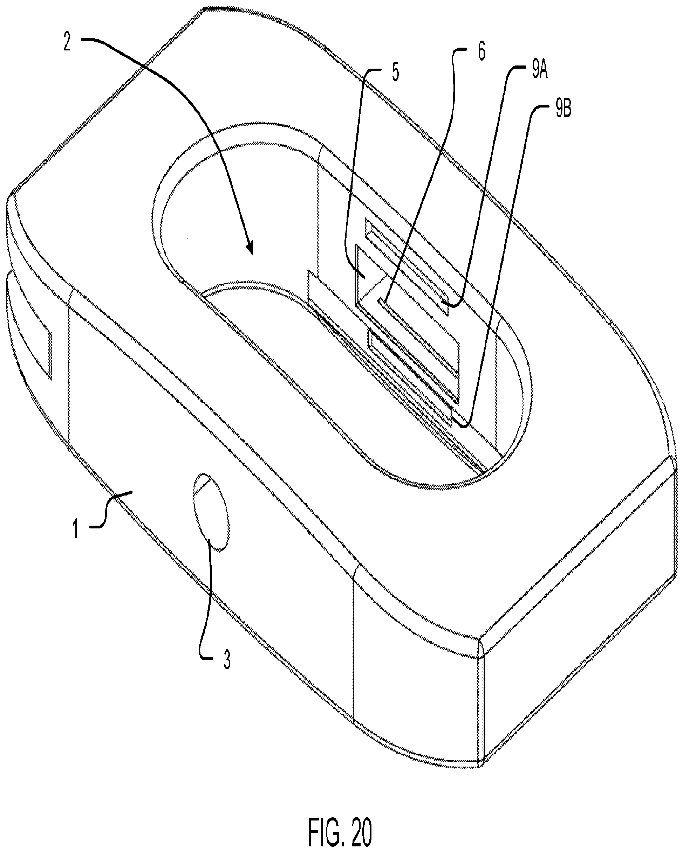

Referring to FIG. 20, implant system 500 may include a cage 1 having a fixation aperture 3 in a first sidewall and a primary sensing slot 5 (also referred to as a primary cavity) in a second sidewall. In the example embodiment, the fixation aperture 3 and sensing slot 5 each extend through a corresponding sidewall of cage 1 from the graft window 2 to the outside. Adjacent to the primary sensing slot 5, an upper secondary sensing slot 9A and a lower secondary sensing slot 9B are disposed adjacent to the primary sensing slot 5. Referring back to FIG. 19, it is shown that the housing 21 may include a sensing protrusion 40 having a size and shape corresponding to a size and shape of the primary sensing slot 5. Similarly, the housing 21 may include an upper secondary protrusion 42A and a lower secondary protrusion 42B having a size and shape corresponding to a size and shape of the upper secondary sensing slot 9A and lower secondary sensing slot 9B, respectively. This arrangement may be particularly advantageous at transmitting stress and strain experienced by cage 1 to the housing 21 and the sensing components therein may thereby have a heightened accuracy of detection.

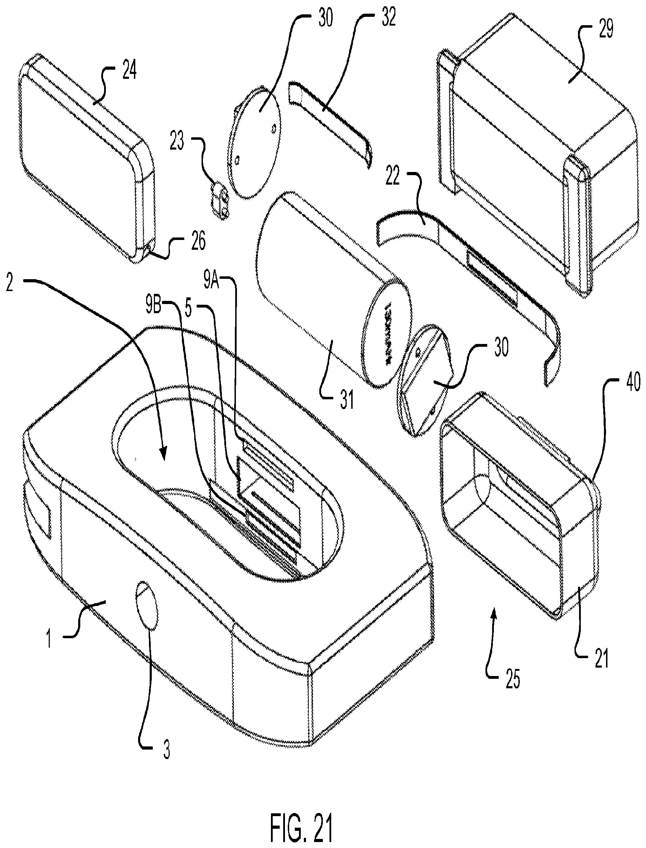

Referring to FIGS. 21-22, a strain gauge 32 may be disposed inside of the cavity 25 in the portion thereof corresponding to the sensing protrusion 40. In this embodiment the strain gauge 32 may have a U-like shape corresponding in size and shape to the sensing protrusion 40. Additionally, in various embodiments the sensing protrusion 40 may include at least one raised rail and/or indented rail portion 41 extending along an outside surface of the sensing protrusion 40. As seen best in FIGS. 20 the primary slotted aperture 5 may include an inferior indented slot 6 and a superior indented slot 7 (not visible from this viewing angle). As explained previously with respect to system 300, the indented slots 6, 7 may correspond in size and shape to the rail portions 41 of the housing 21 such that the rail portions 41 may be disposed inside of slots 6, 7. This configuration of the slotted aperture 5, protruding portion 40, rail portion 41, and slots 6, 7 may be particularly advantageous at accurately transferring stress strain experienced by the cage 1 to the u-shaped strain gauge 32. For example, the U-shaped strain gauge 32 may conform to the shape of the capsule or pill shaped protrusion 40.

In this embodiment, the electronics portion 20 may include a housing 21 defining a cavity 25 therein for housing various electronics components 30. In this embodiment, two distinct electronics components are shown as a circuit board that are in electrical communication with battery 31, strain gauge 32, and antenna 22. The electronics components 30 may have great variability in the types of circuity and hardware due to the relatively large size of the housing 21 and cavity 25. Example electronics components may include a flexible circuit board providing an electrical connection between the battery 31, strain gauge 32, and the various other electronics components. A non-limiting list of example electronics components may include an Application Specific Integrated Controller (ASIC) 34, micro controller 35, a wake-up sensor, a memory storage, an impedance sensor, and a temperature sensor. In at least one embodiment an impedance sensor protrudes into the graft window for assessing the status of a fusion process and a temperature sensor is disposed inside of the cavity 25.

It should be understood that various aspects disclosed herein may be combined in different combinations than the combinations specifically presented in the description and accompanying drawings. For example, features, functionality, and components from one embodiment may be combined with another embodiment and vice versa unless the context clearly indicates otherwise. Similarly, features, functionality, and components may be omitted unless the context clearly indicates otherwise. It should also be understood that, depending on the example, certain acts or events of any of the processes or methods described herein may be performed in a different sequence, may be added, merged, or left out altogether (e.g., all described acts or events may not be necessary to carry out the techniques).

Unless otherwise specifically defined herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an" and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

## Claims

1. A load sensing spinal implant, comprising:
an interbody cage (1) extending in a longitudinal direction from a proximal end (100P) to a distal end (100D) and in a widthwise direction from a first lateral end to a second lateral end;
an electronics portion (20) including a housing (21) defining a sealed cavity (25) for supporting an electronics assembly and a battery (31) therein;
at least one antenna disposed in an antenna housing (22) and in electrical communication with the electronics assembly; and
at least one strain gauge (32) configured to detect a localized force experienced by the interbody cage (1) and being in electrical communication with the electronics assembly,
wherein the at least one antenna is configured to transmit information received from the at least one strain gauge (32) to an external device, and wherein
at least one pass through connection (23) extends through a sidewall of the housing (21) of the electronics portion (20) thereby placing the at least one antenna in electrical communication with the electronics assembly.

2. The load sensing spinal implant of claim 1, wherein the at least one antenna is configured to utilize a Bluetooth low energy (BLE) technology.

3. The load sensing spinal implant of claim 1, wherein the electronics portion (20) is disposed inside of a graft window (2) of the interbody cage (1).

4. The load sensing spinal implant of claim 1, wherein an overmold (29) is formed over the electronics portion (20).

5. The load sensing spinal implant of claim 1, wherein the housing (21) further comprises a cover (24) configured to seal an opening of the cavity (25) for placing the electronics assembly therein.

6. The load sensing spinal implant of claim 1, wherein the electronics assembly is coupled to an exposed side surface of the interbody cage (1).

7. The load sensing spinal implant of claim 1, further comprising at least one of: a temperature sensor, an accelerometer sensor, a gyroscope sensor, and an impedance sensor.

8. The load sensing spinal implant of claim 1, wherein the at least one strain gauge (32) is disposed on an interior sidewall (21A) of a graft window (2) of the interbody cage (1).

9. The load sensing spinal implant of claim 1, wherein the electronics assembly further comprises a wake-up sensor configured to power up the electronics assembly and cause the at least one antenna to initiate a transmission of information to the external device.

10. The load sensing spinal implant of claim 1, wherein the at least one strain gauge (32) is disposed inside of the housing (21).

11. The load sensing spinal implant of claim 1, wherein the electronics portion is disposed inside of a graft window (2) of the interbody cage (1) and the housing (21) comprises a primary protrusion that extends into a first cavity in a side wall of the graft window (2).

12. The load sensing spinal implant of claim 11, wherein the housing (21) comprises a secondary protrusion (42A) that extends into a second cavity in a sidewall of the graft window (2).

13. The load sensing spinal implant of claim 1, wherein the electronics portion (20) is coupled to an interior sidewall (21A) of the interbody cage (1) by a pin or a threaded screw.

14. The load sensing spinal implant of claim 1, wherein the at least one antenna has a size and shape that generally corresponds to at least one interior sidewall (21A) of a graft window (2) of the interbody cage (1).

15. The load sensing spinal implant of claim 1, wherein the interbody cage (1) is formed around the at least one antenna and, when viewed in plan view, the at least one antenna has a size and shape that generally corresponds to a size and shape of the interbody cage (1).

## Patentansprüche

1. Lasterfassendes Wirbelsäulenimplantat, umfassend:
einen Zwischenkörperkäfig (1), der sich in einer Längsrichtung von einem proximalen Ende (100P) zu einem distalen Ende (100D) und in einer Breitenrichtung von einem ersten lateralen Ende zu einem zweiten lateralen Ende erstreckt;
einen Elektronikabschnitt (20) einschließlich eines Gehäuses (21), das einen abgedichteten Hohlraum (25) zum Tragen einer Elektronikanordnung und einer Batterie (31) darin definiert;
mindestens eine Antenne, die in einem Antennengehäuse (22) eingerichtet und mit der Elektronikanordnung elektrisch verbunden ist; und
mindestens einen Dehnungsmesser (32), der konfiguriert ist, um eine lokalisierte Kraft zu erkennen, die auf den Zwischenkörperkäfig (1) wirkt und mit der Elektronikanordnung elektrisch verbunden ist,
wobei die mindestens eine Antenne konfiguriert ist, um Informationen, die von dem mindestens einen Dehnungsmesser (32) empfangen werden, an eine externe Vorrichtung zu übertragen, und wobei
sich mindestens eine Durchgangsverbindung (23) durch eine Seitenwand des Gehäuses (21) der Elektronikanordnung (20) erstreckt und dadurch die mindestens eine Antenne mit der Elektronikanordnung in elektrische Verbindung platziert.

2. Lasterfassendes Wirbelsäulenimplantat nach Anspruch 1, wobei die mindestens eine Antenne konfiguriert ist, um eine Bluetooth Low Energy-Technologie (BLE-Technologie) zu nutzen.

3. Lasterfassendes Wirbelsäulenimplantat nach Anspruch 1, wobei der Elektronikabschnitt (20) innerhalb eines Transplantatfensters (2) des Zwischenkörperkäfigs (1) eingerichtet ist.

4. Lasterfassendes Wirbelsäulenimplantat nach Anspruch 1, wobei eine Umspritzung (29) über dem Elektronikabschnitt (20) ausgebildet ist.

5. Lasterfassendes Wirbelsäulenimplantat nach Anspruch 1, wobei das Gehäuse (21) ferner eine Abdeckung (24) umfasst, die konfiguriert ist, um eine Öffnung des Hohlraums (25) zum Platzieren der Elektronikanordnung darin abzudichten.

6. Lasterfassendes Wirbelsäulenimplantat nach Anspruch 1, wobei die Elektronikanordnung an einer freiliegenden Seitenoberfläche des Zwischenkörperkäfigs (1) gekoppelt ist.

7. Lasterfassendes Wirbelsäulenimplantat nach Anspruch 1, ferner umfassend mindestens einen von: einem Temperatursensor, einem Beschleunigungssensor, einem Gyroskopsensor und einem Impedanzsensor.

8. Lasterfassendes Wirbelsäulenimplantat nach Anspruch 1, wobei der mindestens eine Dehnungsmesser (32) an einer inneren Seitenwand (21A) eines Transplantatfensters (2) des Zwischenkörperkäfigs (1) eingerichtet ist.

9. Lasterfassendes Wirbelsäulenimplantat nach Anspruch 1, wobei die Elektronikanordnung ferner einen Wecksensor umfasst, der konfiguriert ist, um die Elektronikanordnung mit Leistung zu versorgen und die mindestens eine Antenne zu veranlassen, eine Informationsübertragung an die externe Vorrichtung einzuleiten.

10. Lasterfassendes Wirbelsäulenimplantat nach Anspruch 1, wobei der mindestens eine Dehnungsmesser (32) innerhalb des Gehäuses (21) eingerichtet ist.

11. Lasterfassendes Wirbelsäulenimplantat nach Anspruch 1, wobei der Elektronikabschnitt innerhalb eines Transplantatfensters (2) des Zwischenkörperkäfigs (1) eingerichtet ist und das Gehäuse (21) einen primären Vorsprung aufweist, der sich in einen ersten Hohlraum in einer Seitenwand des Transplantatfensters (2) erstreckt.

12. Lasterfassendes Wirbelsäulenimplantat nach Anspruch 11, wobei das Gehäuse (21) einen sekundären Vorsprung (42A) aufweist, der sich in einen zweiten Hohlraum in einer Seitenwand des Transplantatfensters (2) erstreckt.

13. Lasterfassendes Wirbelsäulenimplantat nach Anspruch 1, wobei der Elektronikabschnitt (20) mittels eines Stifts oder einer Gewindeschraube mit einer inneren Seitenwand (21A) des Zwischenkörperkäfigs (1) gekoppelt ist.

14. Lasterfassendes Wirbelsäulenimplantat nach Anspruch 1, wobei die mindestens eine Antenne eine Größe und Form aufweist, die im Allgemeinen mindestens einer inneren Seitenwand (21A) eines Transplantatfensters (2) des Zwischenkörperkäfigs (1) entspricht.

15. Lasterfassendes Wirbelsäulenimplantat nach Anspruch 1, wobei der Zwischenkörperkäfig (1) um die mindestens eine Antenne herum ausgebildet ist und die mindestens eine Antenne in der Draufsicht eine Größe und Form aufweist, die im Allgemeinen einer Größe und Form des Zwischenkörperkäfigs (1) entspricht.

## Revendications

1. Implant rachidien à détection de charge, comprenant :
une cage intersomatique (1) s'étendant dans un sens longitudinal d'une extrémité proximale (100P) à une extrémité distale (100D) et dans un sens de la largeur d'une première extrémité latérale à une seconde extrémité latérale ;
une partie électronique (20) comportant un boîtier (21) définissant une cavité scellée (25) pour supporter un ensemble électronique et une batterie (31) à l'intérieur de celle-ci ;
au moins une antenne disposée dans un boîtier d'antenne (22) et en communication électrique avec l'ensemble électronique ; et
au moins une jauge de contrainte (32) configurée pour détecter une force localisée subie par la cage intersomatique (1) et étant en communication électrique avec l'ensemble électronique,
dans lequel l'au moins une antenne est configurée pour transmettre les informations reçues de l'au moins une jauge de contrainte (32) à un dispositif externe, et dans lequel
au moins une connexion de passage (23) s'étend à travers une paroi latérale du boîtier (21) de la partie électronique (20), plaçant ainsi l'au moins une antenne en communication électrique avec l'ensemble électronique.

2. Implant rachidien à détection de charge selon la revendication 1, dans lequel l'au moins une antenne est configurée pour utiliser une technologie Bluetooth low energy (BLE).

3. Implant rachidien à détection de charge selon la revendication 1, dans lequel la partie électronique (20) est disposée à l'intérieur d'une fenêtre de greffe (2) de la cage intersomatique (1).

4. Implant rachidien à détection de charge selon la revendication 1, dans lequel un surmoulage (29) est formé sur la partie électronique (20).

5. Implant rachidien à détection de charge selon la revendication 1, dans lequel le boîtier (21) comprend en outre un couvercle (24) conçu pour sceller une ouverture de la cavité (25) afin de placer l'ensemble électronique à l'intérieur de celle-ci.

6. Implant rachidien à détection de charge selon la revendication 1, dans lequel l'ensemble électronique est couplé à une surface latérale exposée de la cage intersomatique (1).

7. Implant rachidien à détection de charge selon la revendication 1, comprenant en outre au moins l'un parmi : un capteur de température, un capteur d'accéléromètre, un capteur de gyroscope et un capteur d'impédance.

8. Implant rachidien à détection de charge selon la revendication 1, dans lequel l'au moins une jauge de contrainte (32) est disposée sur une paroi latérale intérieure (21A) d'une fenêtre de greffe (2) de la cage intersomatique (1).

9. Implant rachidien à détection de charge selon la revendication 1, dans lequel l'ensemble électronique comprend en outre un capteur de réveil configuré pour mettre l'ensemble électronique sous tension et amener l'au moins antenne à initier une transmission d'informations vers le dispositif externe.

10. Implant rachidien à détection de charge selon la revendication 1, dans lequel l'au moins une jauge de contrainte (32) est disposée à l'intérieur du boîtier (21).

11. Implant rachidien à détection de charge selon la revendication 1, dans lequel la partie électronique est disposée à l'intérieur d'une fenêtre de greffe (2) de la cage intersomatique (1) et le boîtier (21) comprend une protubérance primaire qui s'étend dans une première cavité dans une paroi latérale de la fenêtre de greffe (2).

12. Implant rachidien à détection de charge selon la revendication 11, dans lequel le boîtier (21) comprend une protubérance secondaire (42A) qui s'étend dans une seconde cavité dans une paroi latérale de la fenêtre de greffe (2).

13. Implant rachidien à détection de charge selon la revendication 1, dans lequel la partie électronique (20) est couplée à une paroi latérale intérieure (21A) de la cage intersomatique (1) par une broche ou une vis filetée.

14. Implant rachidien à détection de charge selon la revendication 1, dans lequel l'au moins une antenne a une taille et une forme qui correspondent généralement à au moins une paroi latérale intérieure (21A) d'une fenêtre de greffe (2) de la cage intersomatique (1).

15. Implant rachidien à détection de charge selon la revendication 1, dans lequel la cage intersomatique (1) est formée autour de l'au moins une antenne et, vue en plan, l'au moins une antenne a une taille et une forme qui correspondent généralement à la taille et à la forme de la cage intersomatique (1).
